# EUROPEAN PATENT APPLICATION

(11) **EP 2 689 729 A1**
(43) Date of publication of application: **29.01.2014**
(21) Application number: 12177471.5
(22) Date of filing: 23.07.2012
(51) Int. Cl.: A61B 10/00, G01N 33/48, B01L 3/00

(54) **Disposable test device**

(71) Applicant: DML - ABLogics Limited, London SW5 9DB (GB)
(72) Inventor: Catteruccia, Nicoletta, London SW5 9DB (GB); Friedlander, Uri, London NW3 4AU (GB)
(74) Representative: Minoja, Fabrizio

(57) **Abstract**

A disposable test device for detecting the presence of an analyte in a liquid sample derived from biological specimen, e.g. saliva, urine, blood or the like, comprising:
- a reagent container (10),
- a test chamber (20), and
- a test pill (30) of absorbent material,

wherein the test chamber contains at least a test strip (26), and the reagent container (10) can be mated with the test chamber (30) with the test pill (20) sandwiched between the regent container and the test chamber, such that the reagent moves a sample which is impregnated in the test pill towards the test strip (26).

## Description

The present invention relates to a disposable test device for detecting a target an analyte in a liquid sample derived from biological specimen, e.g. saliva, urine, blood or the like. In particular, the invention relates to test devices in form of test strips suited for carrying out lateral flow immunoassays.

### Background of the invention

Immunoassays are widely used to detect a substance of diagnostic interest in a sample by means of the binding of an antibody to its antigen. Monoclonal antibodies are preferred in view of their specificity and selectivity. Immunoassays are routinely used, for example, for the diagnosis of a number of disease including HIV, influenza, hepatitis B or C, autoimmune diseases, neoplasias, etc., as well as for measuring any analyte against which an antibody-antigen reaction may be established. Examples of said analytes include hormones, drugs, markers, proteins, and the like.

Labelled antibodies or antigens are generally used in immunoassays to give a signal of the presence of a given analyte in the sample. The label is mostly conjugated to an antibody that binds to the analyte. The type of label used may vary, and may include visually detectable labels as well as labels that require instrumentation for detection. Examples of said labels include colloidal gold, enzymes, radioisotopes, fluorescent molecules or other chromophores.

A particularly convenient format for immunoassays is provided by the so-called lateral flow or flow through assays disclosed for instance in US 5,714,389 and in US 5,989,921.

In lateral flow assays, a liquid containing an analyte of interest migrates by capillary force along a membrane strip having suitable reagents impregnated thereon. The liquid sample, applied to one end of the strip, is eluted to a detection zone where a second antibody, immobilized on the strip, allows the visualization of the diagnostic test. Lateral flow assays may be easily carried out in a short time even in non specialized environments and are characterised by high accuracy and sensitivity.

Lateral flow assays may be of the "competitive" or "non-competitive" type. In the competitive-type immunoassays, analyte in a sample is mixed with analyte conjugated to a detectable label. The mixture then migrates along a membrane where the analyte in the sample and the labelled analyte compete for binding to a binding agent specific for the analyte immobilized on the test strip. The amount of labelled analyte detected at the detection zone is inversely proportional to the concentration of analyte in the sample. In "non-competitive", also known as"sandwich"-type immunoassays, the analyte in the sample binds to a first binding reagent conjugated to a label. The sample containing the complex analyte-labelled binding reagent antigen is then contacted with a test strip on which the complex binds to a second binding reagent immobilized on the membrane. In correspondence of the zone where the complex is captured by the second binding reagent, a visual signal appears. Several devices for lateral flow immunoassays have been disclosed. They typically employ different absorbent materials and a membrane, usually a nitrocellulose membrane. Lateral flow devices may be configured so as to have the labelled reagent adsorbed on the test strip so that no further reagent or eluent is required, the liquid sample acting as an eluent. This kind of devices may be convenient when the liquid sample is easy to collect and available in large amounts, for instance in the case of pregnancy tests to be carried out on urine. For other types of assays, an eluent such as a buffer is required. The labelled reagent, instead of being adsorbed on the strip, may also be added to the liquid sample prior to contacting the sample to the test strip.

The assays requiring a separate liquid reagent, such as a buffer system, may be difficult to perform outside an equipped laboratory, by non trained users who are required to measure precise volumes of reagent to be applied on the test strip. In order to overcome this drawback, a number of devices have been proposed wherein a portion of the test strip is configured so as to form a container or reservoir containing the liquid reagent, with the purpose of avoiding the operations of sampling and measuring the liquid reagent. The known devices are however cumbersome since the container formed on the test strip is liable to leakage of the fluid contained therein, as a consequence of the geometry intrinsic to the test strip itself.

Most known lateral flow assays have to be directly contacted with the physiological fluid (blood, saliva, urine, etc) and this may be again a potential source of problems for inexpert users.

### Description of the invention

An object of the invention is to provide a disposable test device free from the above mentioned drawbacks of the known devices and which is simple to use by personnel with no specialized training, has a simple and inexpensive construction and is suitable to make different tests simultaneously on a bodily fluid.

Another object of the invention is to provide a disposable test device allowing an easy sampling of the physiological fluid containing the analyte of interest, increasing thereby the test accuracy and sensitivity, allowing the fluid to be concentrated on a separate member of the device suited also for *in vivo* diagnostic procedures.

These objects are achieved by the disposable test device according to the present invention which comprises the features listed in the appended independent claim 1.

The invention relates also to a method of operation of such a test device, according to claim 9.

Preferred embodiments of the invention are disclosed in the dependent claims.

Essentially, the disposable test device according to the invention comprises: a reagent container, a test chamber and a test pill.

The reagent container is in the form of a cylinder open at one end, wherein a perforated piston of elastic material, such as rubber, can slide. The open end of the container can be closed by a sealing foil to prevent any potential loss of reagent.

The test chamber comprises an outer tubular housing, an inner support member and at least one, preferably a plurality of test strips interposed between the inner support member and the outer housing.

During use, the test pill, which is in absorbent material, previously impregnated with bodily fluid, is sandwiched between the test chamber and the reagent container, from which the sealing foil has been removed, and the bodily fluid mixed with the reagent is transferred to the test strips.

The test pills are particularly convenient for testing analytes contained in the saliva since they may be ingested by the subjects in need of a diagnosis and kept in the mouth for a period of time sufficient to capture the analyte. This test would be particularly convenient, for instance, in the diagnosis of influenza type A and/or type B infections where the viral antigens are present in minute amounts in the saliva of infected subjects. Malaria and other infective disease may also be effectively diagnosed by means of the devices of the invention. Other examples of analytes which may be conveniently detected in the saliva or other physiological fluids (e.g. sweat) include drugs of abuse (cocaine, heroin, amphetamines, barbiturates, benzodiazepines, methadone) and anabolising steroids.

The pills may consist of any suitable absorbent material, typically synthetic, semi-synthetic or natural polymers. Preferred materials include cellulose derivatives such as ethylcellulose, hydroxyethylcellulose, hydroxypropylcellulose, in admixtures with lubricants, tabletting agents, surfactants and other conventional excipients.

The size of the pill is not critical and will depend on the specific test for which it has been designed. Anyhow, a length of 10-15 mm and a few mm of diameter will be suited for most applications.

Further features of the invention will appear from the following detailed description of purely illustrative and therefore not limitative embodiments thereof made in conjunction with the attached drawings, wherein:
Fig. 1 is a diagrammatic longitudinal sectional view of a disposable test device according to the invention, showing the reagent container, the test pill and the test chamber before assembly;
Fig. 2 is a perspective exploded enlarged view of the reagent container;
Fig. 3 is a perspective view of the test chamber;
Fig. 4 is a longitudinal section through the assembled test chamber;
Fig. 5 a) - f) are diagrammatic sectional views showing successive steps during operation of the device;
Fig. 6 and 7 are diagrammatic perspective exploded views showing two possible shapes of the test chamber.
Fig. 8 to 10 show a different embodiment of the test chamber of the device according to the invention; in particular: Fig. 8 is an perspective view; Fig. 9 is a longitudinal section of the device before assembly; Fig. 10 is a longitudinal section of the device after test has been completed.

In the following description similar reference numerals refer to similar parts in the different drawings.

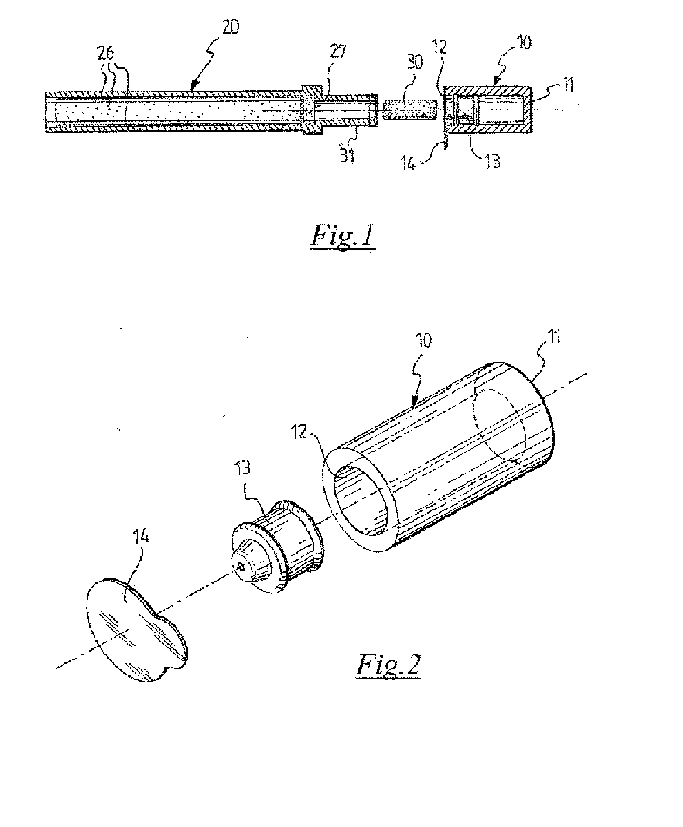

Referring to Fig. 1 of the drawings, the disposable test device according to the invention comprises:
- a reagent container 10,
- a test chamber 20, and
- a test pill 30.

The reagent container 10 (see in particular Fig. 2), preferably in transparent rigid plastic material, is in the form of a cylinder, with a closed end 11 and an open end 12. A rubber piston 13 can be inserted through the open end 12 and slide within the cylinder container 10. For filling the container 10 with the reagent, the piston is pushed to the bottom of the container, towards the closed end 11. Then, a needle of a syringe (not shown), which contains the test reagent, is inserted into the rubber piston all the way to the bottom of the container. The syringe is activated and the reagent fills the container 10 while pushing the piston towards the open end 12.

After the container 10 has been filled until the top of the piston is level with open end 12 of the container, the syringe is extracted and a sealing foil 14 is heat stacked onto the open end of the container preventing any potential loss of reagent in storage.

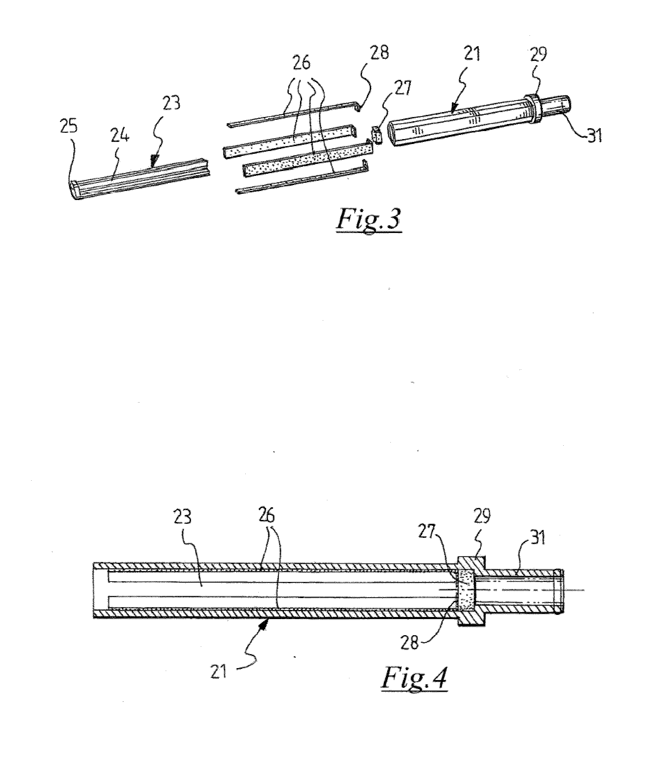

Referring now to Fig. 3, the test chamber 20 is assembled from four main parts:
- an outer tubular housing 21 preferably molded in transparent rigid plastic material,
- an inner support member 23, also molded in rigid plastic material, having on its outer surface a plurality of longitudinal grooves 24 and a distal radially projecting head 25,
- a plurality of test strips 26 able to be housed in said longitudinal grooves 24 such as to be interposed between the inner support member 24 and the outer housing 21, and
- an absorbent pad 27 cut or sintered from absorbent material, inserted in the proximal end 29 of the outer housing.

The proximal end 28 of the test strips 26 is inwardly folded about 90° such that after being positioned on the inner support member 23 and inserted into the outer housing 21, they are pushed to form an intimate contact with the absorbent pad 27.

From the proximal end 29 of the outer housing 21 projects a short tubular portion 31, preferably of circular section, having a diameter less than the outer contour of the housing 21 and such as to tightly slide into the reagent container 10.

The test pill 30 is formed of absorbent material which can be used orally or in any other suitable way.

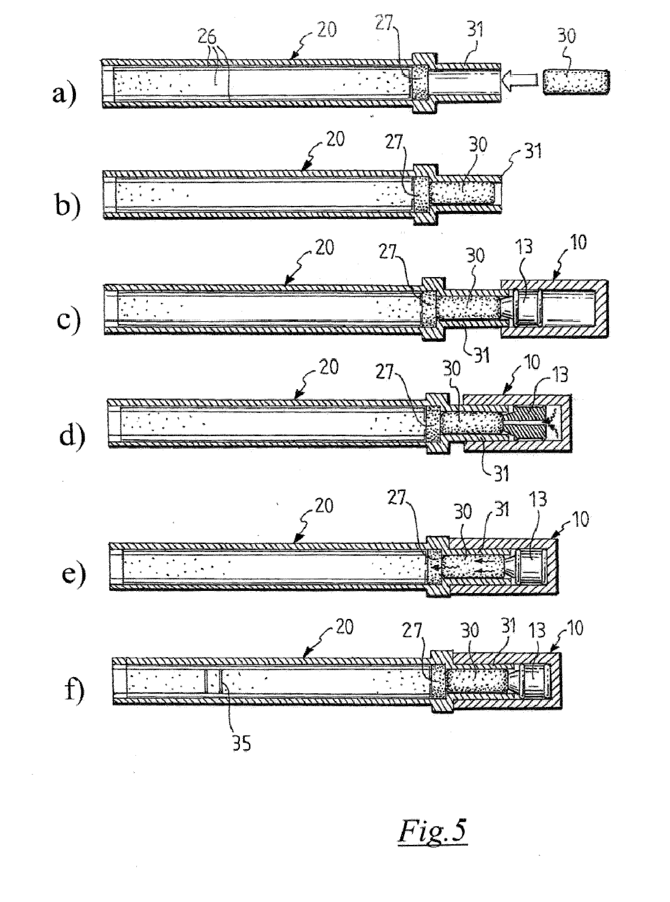

Referring now to FIgg. 5 a) to f), the operation of the device will be described.

The pill 30 is submerged in the sample and inserted into the tubular portion 31 of the test chamber until it forms an intimate contact with the absorbent pad 27 (Figg 5 a) and b)).

Thereafter, the sealing foil 14 is removed from the reagent container 10 and the reagent container is mated with the tubular portion 31 of the test chamber (Fig. 5 c)).

The piston 13 inside the reagent container 10 is pushed backwards by the tubular portion 31 and the reagent moves towards the test pill 30 through the central hole previously made by the syringe needle in the piston 13 (Fig. 5 d)).

The reagent pushes through and over the test pill 30 and moves the sample which is impregnated in the test pill towards the absorbent pad 27 (Fig. 5 e)).

The capillary test strips 26 transport the test reagent through them and a detection signal line 35 which is printed on the test strips becomes visible.

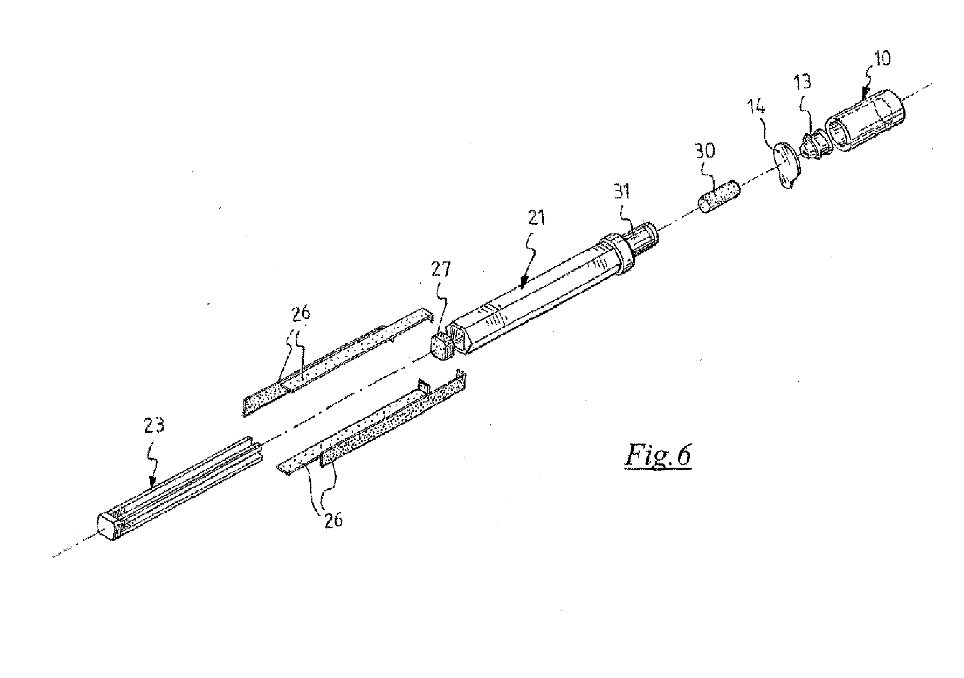

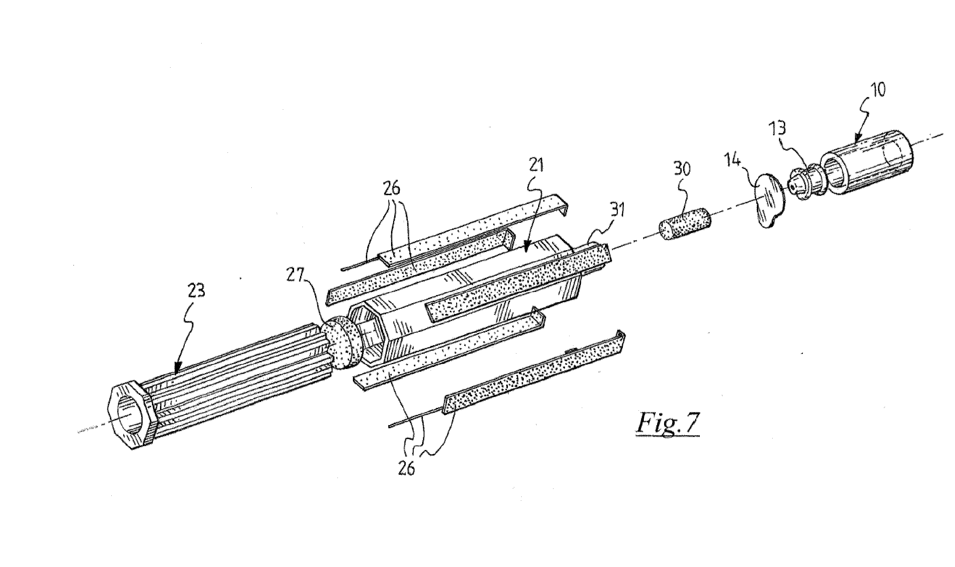

The device of the invention, in particular the test chamber 20 can be of any suitable shape. By way of example only, the embodiments of figures 6 and 7 show the test chamber 20 of square and octagonal cross-section, respectively. Of course the test chamber could also be of substantially circular cross-section.

The reagent chamber also could be of any convenient shape, although it is preferred a circular cross-section for a better sliding of the piston 13 therein.

In the foregoing description, the terms "proximal" and "distal" are referred to the right part of the drawings, i.e. with respect to the reagent chamber 10.

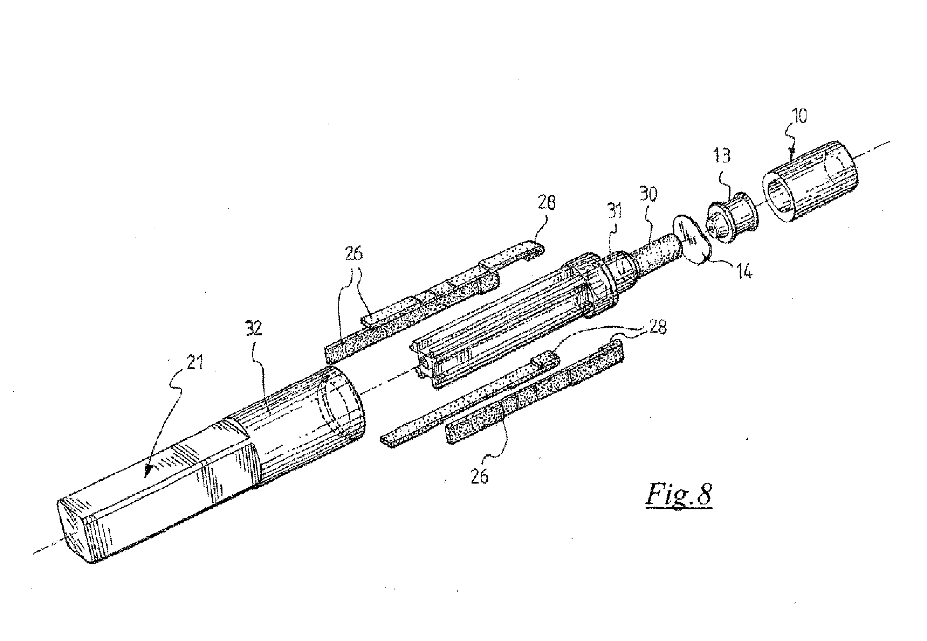

Figures 8 to 10 show the device of the invention with a different embodiment of the test chamber with respect to that shown in particular in Fig. 3. In such figures the same reference numerals as in the previous embodiment are used for indicating the same or similar parts.

According to this embodiment, the tubular portion 31 projects from the proximal end of the inner support member 23 and the proximal end 28 of the test strip 26 is inwardly folded about 180° to be in direct contact with the test pill 30 when the device is assembled, without any extra absorbent pad between the test pill 30 and the test strips 26.

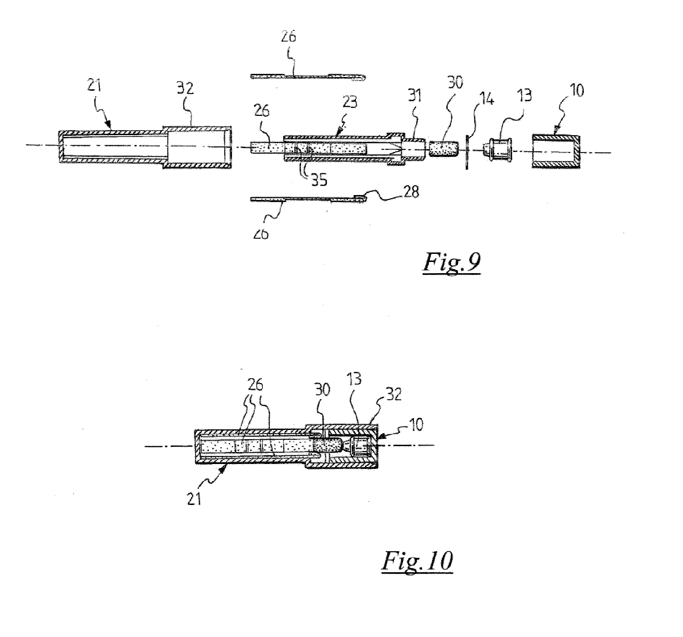

The outer housing 21 has the distal end closed wherein a vent hole is provided and the proximal end opened for insertion of the inner support member 23. An enlarged tubular portion 32 projects from the proximal end of the outer housing 21 to cover the reagent container 10 when it is inserted on the tubular portion31 of the inner support member 23 (Fig. 10).

The following examples illustrate typical tests which can be carried out with the device according to the invention.

### Example 1

### Influenza A And B Test Detection

Influenza test involves the extraction of influenza A and B viral antigen. The specimen is collected by a sterile pill which is kept in the mouth for 5 min. The viral antigens is adsorbed on the sterile pill. The strip is contained in a device cassette.

An antibody specific for the target of interest (anti NP A, anti NPB, HA or NA) is immobilized on a nitrocellulose membrane in the form of a line, called the test line.

A secondary antibody against the primary antibody, generally an anti mouse IgG, is also immobilized on the nitrocellulose membrane in the form of a line called the control line.

Gold nanoparticles conjugated with analytical specific antibody are pre adsorbed into the conjugated pad.

The pill is then placed onto the housing (just over the sample pad strip) and after the contact with the specific extraction buffer, the virus particles are disrupted. Through capillary action, the antigens (NPs, HA and NA) are transported into the conjugate pad, where bind gold nanoparticles antibody conjugated (GNP-mAb).

On the test line the anti influenza antibody catches the GNP- mAb coated with antigens, while the second antibody (anti mouse IgG) catches particles which did not bind to an analyte on the control line.

If influenza A or B antigens are present will appear a specific red line together with a positive control line, if influenza antigens are not present will appear just the positive control line.

This immunoassay format described above, can be also applied for a malaria lateral flow test. As the test sample (blood or serum) flows through the membrane the antigen HRP-2, or other specific malaria proteins, bind the gold nanoparticles monoclonal antibody conjugated.

This complex moves further on the membrane to the test region where it is immobilised by the anti HRP-2 (monoclonal) coated on the membrane.

The formation of a colored band confirms a positive test result. The absence of the colored band in the test region indicates a negative test result. The unreacted conjugate move and subsequently immobilised by anti mouse antibodies coated on the membrane at the control region, forming a red band.

### Example 2

### Drug Of Abuse Test Detection

### Competitive immunoassay: when the target analytes consist of small molecules, as in the case of drugs of abuse, competitive assays are often preferred

After the extraction of the sample form the pill the analyte are transported into the conjugate pad.The conjugate pad contains antibodies that are already bound to an analogue of the target analyte.

If the target analyte is present in the sample it will therefore not bind with the conjugate and will remain unlabelled. As the sample migrates along the membrane and reaches the capture zone an excess of unlabelled analyte will bind to the immobilised antibodies and block the capture of the conjugate, so that no visible line is produced. The unbound conjugate will then bind to the antibodies in the control zone producing a visible control line. A single control line on the membrane is a positive result. Two visible lines in the capture and control zones is a negative result.

Of course the invention is not restricted to the embodiments previously described and shown in the appended drawing, instead many modifications and changes can be made, falling within the scope of protection defined by the appended claims.

## Claims

1. A disposable test device for detecting the presence of an analyte in a liquid sample derived from biological specimen, e.g. saliva, urine, blood or the like, comprising:
- a reagent container (10),
- a test chamber (20), and
- a test pill (30) of absorbent material,
wherein the test chamber contains at least a test strip (26), and the reagent container (10) can be mated with the test chamber (20) with the test pill (30) sandwiched between the reagent container and the test chamber, such that the reagent moves a sample which is impregnated in the test pill towards said at least a test strip (26).

2. Test device according to claim 1, wherein said reagent container (10) is in the form of a cylinder wherein a perforated piston (13) of elastic material can slide, the container (10) being suitable to be inserted on a tubular projection (31) of the test chamber wherein said test pill (30) is housed.

3. Test device according to claim 2, wherein a sealing foil (14) is heat stacked onto the container (10) to prevent any loss of reagent during storage.

4. Test device according to claim 1, wherein said test chamber comprises an outer tubular housing (21) and an inner support member (23) having a plurality of longitudinal grooves (24), said test strips (26) being suitable to be housed in said longitudinal grooves (24) and interposed between the inner support member (23) and the outer housing (21).

5. Test device according to claim 4, wherein said test strips (26) have their proximal end (28) inwardly folded about 180° to be in intimate contact with said test pill (30).

6. Test device according to claim 4, wherein said test strips (26) have their proximal end (28) inwardly folded about 90° to be in intimate contact with an absorbent pad (27) in turn in contact with said test pill (30).

7. Test device according to anyone of the preceding claims, wherein said reagent container (10) and said test chamber (20) are made of transparent, rigid, plastic material.

8. Test device according to anyone of the preceding claims, wherein said test chamber (20) has a circular or polygonal cross-section.

9. Test device according to anyone of the preceding claims suitable for making different tests simultaneously on the same sample.

10. Method of operation of the test device according to anyone of the preceding claims, comprising the following steps:
- insertion of the test pill (30) impregnated with a liquid sample into the tubular projection (31) of the test chamber (20) until it forms an intimate contact with the test strips (26) or the absorbent pad (27);
- removal of the sealing foil (14) from the reagent container (10) and mating the reagent container with the tubular portion (31) of the test chamber (20);
- pushing backwards the piston (13) inside the reagent container (10) through the tubular portion (31) so that the reagent moves towards the test pill (30) through a central hole in the piston (13);
- the reagent pushes through and over the test pill (30) and moves the sample which is impregnated in the test pill towards the test strips (26) or the absorbent pad (27) in contact therewith;
- the capillary test strips (26) transport the test reagent through them and a detection signal line (35) which is printed on the test strips becomes visible.

11. Method according to claim 10, wherein said reagent container (10) is filled with the reagent by pushing the piston (13) to the bottom of the container; inserting a needle of a syringe, which contains the test reagent into the piston (13) all the way to the bottom of the container; activating the syringe so that the reagent fills the container (10) while pushing the piston (13) towards the opening of the container.
